# EUROPEAN PATENT APPLICATION

(11) **EP 4 850 602 A1**
(43) Date of publication of application: **09.12.2026**
(21) Application number: 25747562.4
(22) Date of filing: 16.01.2025
(51) Int. Cl.: A61B 5/145

(54) **INTEGRATED PRESS-TRIGGERED APPLICATION DEVICE**

(30) Priority: 02.02.2024 CN 202420279540 U
(71) Applicant: SHENZHEN SISENSING CO., LTD., Shenzhen Guangdong 518110 (CN)
(72) Inventor: LI, Yunfeng, Shenzhen, Guangdong 518110 (CN)
(74) Representative: Vossius & Partner Patentanwälte Rechtsanwälte mbB
(86) International application number: PCT/CN2025/072870
(87) International publication number: WO 2025/161990

(57) **Abstract**

Disclosed is a unitary press-triggered applicator for applying a medical device to a subject. The applicator includes a first housing, a second housing, and a movable assembly, together with a proximal end adjacent to the subject and a distal end away from the subject during operation. The first housing is assembled to the second housing, and the movable assembly, which is assembled to the second housing and movable relative thereto from the distal end toward the proximal end, includes a receiving portion configured to accommodate the medical device, a retaining mechanism releasably retained by the housings, and a guide member configured to guide application of the medical device. When the first housing moves relative to the second housing toward the proximal end, the housings release the retaining mechanism.

## Description

### BACKGROUND OF INVENTION

The present disclosure relates to the technical field of biomedical engineering, and in particular, to a unitary press-triggered applicator.

### DESCRIPTION OF RELATED ART

For diabetic subjects, continuous real-time monitoring of glucose concentration in body fluids is necessary in daily life, so that subjects can timely adjust the glucose concentration through diet regulation or medication administration, etc., thereby reducing the occurrence of complications caused by abnormal glucose concentration. Conventional methods for monitoring glucose concentration in body fluids require repeated finger-prick blood sampling by users, which is not only cumbersome but also causes significant discomfort to subjects. A Continuous Glucose Monitoring (CGM) device is an apparatus for dynamic monitoring of human glucose concentration, which does not require repeated finger-prick blood sampling.

Before a CGM device starts normal operation, it needs to be applied to the skin surface of a human body first. In the prior art, a special applicator is used to pick up the CGM device, and the CGM device is applied to the skin surface of the human body by pressing a button on the housing of the applicator, thereby realizing continuous dynamic monitoring of glucose concentration in human body fluids.

However, the applicator in the prior art always triggers the action of applying the CGM device to the skin surface of the human body by pressing the button. Due to the small force application area of the button, the triggering mode of the applicator is still not convenient enough.

### SUMMARY OF INVENTION

The present disclosure is proposed in view of the above circumstances, and it is an objective of the present disclosure to provide a unitary press-triggered applicator capable of providing improved actuation convenience of a triggering mode.

To this end, the present disclosure provides a unitary press-triggered applicator for applying a medical device to a subject, which includes: a first housing, a second housing, a movable assembly, a proximal end adjacent to the subject during operation, and a distal end away from the subject during operation, where the first housing is assembled to the second housing and the movable assembly is assembled to the second housing, the movable assembly, which is movable relative to the second housing from the distal end toward the proximal end, includes a receiving portion configured to accommodate the medical device, a retaining mechanism releasably retained by the first housing and the second housing, and a guide member configured to guide application of the medical device; and the first housing and the second housing release the retaining mechanism when the first housing moves relative to the second housing from the distal end toward the proximal end.

In the present disclosure, movement of the first housing relative to the second housing from the distal end toward the proximal end enables the first housing and the second housing to release the retaining mechanism. In other words, pressing the first housing enables the movable assembly to move relative to the second housing from the distal end toward the proximal end to guide the medical device to be applied to the subject, thereby providing improved actuation convenience of the triggering mode.

In addition, in the unitary press-triggered applicator according to the present disclosure, optionally, the applicator further includes a first driving mechanism disposed between the first housing and the movable assembly, where the first driving mechanism applies to the movable assembly an acting force toward the proximal end. In this case, after the first housing and the second housing release the retaining mechanism, the acting force applied by the first driving mechanism to the movable assembly can drive the movable assembly to move toward the proximal end, thereby facilitating proximal movement of the medical device by the movable assembly.

In addition, in the unitary press-triggered applicator according to the present disclosure, optionally, the first housing defines a first limiting mechanism, and the second housing defines a mating portion slidably engaged with the first limiting mechanism, such that movement of the first housing relative to the second housing from the distal end toward the proximal end can be facilitated.

In addition, in the unitary press-triggered applicator according to the present disclosure, optionally, the retaining mechanism includes a first retaining portion, the first housing defines a second limiting mechanism, and the first retaining portion is releasably retained by the second limiting mechanism, such that the retaining mechanism can be releasably retained by the first housing.

In addition, in the unitary press-triggered applicator according to the present disclosure, optionally, the retaining mechanism includes a first retaining arm that flares outwardly in a direction from the proximal end toward the distal end, and a second retaining portion releasably retained by the second housing; the second retaining portion and the first retaining portion are sequentially formed on the first retaining arm in the direction from the proximal end toward the distal end. In this case, when the second retaining portion shifts toward a central axis of the movable assembly (for example, when the second retaining portion is subjected to an acting force toward the central axis of the movable assembly), the shifted second retaining portion can shift the first retaining arm toward the central axis of the movable assembly, such that the shifted first retaining arm drives the first retaining portion to shift toward the central axis of the movable assembly, thereby enabling the second limiting mechanism to release the first retaining portion, thereby facilitating releasing the movable assembly by the first housing and the second housing.

In addition, in the unitary press-triggered applicator according to the present disclosure, optionally, the second housing includes a third limiting mechanism abutting against the second retaining portion, and the first retaining portion abuts against the third limiting mechanism after the first retaining portion is released from the first housing, such that the risk that the released movable assembly is detached from the second housing can be reduced.

In addition, in the unitary press-triggered applicator according to the present disclosure, optionally, the movable assembly has a first bottom portion proximate to the distal end, a second bottom portion proximate to the proximal end, and a side wall connecting the first bottom portion and the second bottom portion; the first driving mechanism is disposed between the first housing and the first bottom portion, such that the first driving mechanism can act on the movable assembly by acting on the first bottom portion, and structural compactness of the movable assembly can be improved.

In addition, in the unitary press-triggered applicator according to the present disclosure, optionally, the movable assembly has an opening proximate to the distal end, a second bottom portion proximate to the proximal end, and a side wall connecting the opening and the second bottom portion; the side wall includes an extension section extending along a central axis of the movable assembly toward an outer periphery of the movable assembly; the first driving mechanism is disposed between the first housing and the extension section, such that the first driving mechanism can act on the movable assembly by acting on the extension section, and structural flexibility of the movable assembly can be improved.

In addition, in the unitary press-triggered applicator according to the present disclosure, optionally, the guide member is releasably retained by the side wall, a second driving mechanism is disposed between the guide member and the second bottom portion, and the second driving mechanism applies to the guide member an acting force toward the distal end. In this case, when the guide member is released from the side wall, the acting force applied by the second driving mechanism to the guide member can drive the guide member to move toward the distal end, such that the guide member can be conveniently decoupled from the medical device after the guide member guides the medical device to be applied to the subject, thereby improving operation convenience of the applicator.

In addition, in the unitary press-triggered applicator according to the present disclosure, optionally, the guide member includes a second retaining arm releasably retained by the side wall; the second retaining arm flares outwardly in the direction from the proximal end toward the distal end and abuts against the side wall; the second housing defines a triggering mechanism, and the triggering mechanism acts on the second retaining arm during movement of the movable assembly toward the proximal end. In this case, the triggering mechanism acting on the second retaining arm can shift the second retaining arm toward the central axis of the movable assembly, such that the second retaining arm disengages from the side wall, thereby enabling the guide member to move toward the distal end under the action applied by the second driving mechanism, such that decoupling of the guide member from the medical device can be facilitated.

The present disclosure may provide a unitary press-triggered applicator with improved actuation convenience of the triggering mode.

### BRIEF DESCRIPTION OF THE DRAWINGS

The present disclosure will now be explained in further detail by way of example only with reference to the accompanying drawings.
FIG. 1 is a schematic diagram illustrating an assembly of an applicator and a cartridge assembly according to an example of the present disclosure.
FIG. 2 is a schematic diagram illustrating separation of an applicator and a cartridge assembly according to an example of the present disclosure.
FIG. 3 is an exploded schematic diagram of an applicator and a cartridge assembly according to an example of the present disclosure.
FIG. 4 is a schematic diagram illustrating an assembly of a first housing and a second housing according to an example of the present disclosure.
FIG. 5 is a cross-sectional view taken along section line AA' in FIG. 4 according to an example of the present disclosure.
FIG. 6 is an exploded schematic diagram of a movable assembly according to an example of the present disclosure.
FIG. 7 is a cross-sectional view of an applicator taken along a plane perpendicular to section line AA' in FIG. 4 according to an example of the present disclosure.
FIG. 8 is a schematic cross-sectional view illustrating cooperation between a guide member and a side wall according to an example of the present disclosure.

### DETAILED DESCRIPTION

Hereinafter, preferred embodiments of the present disclosure will be described in detail with reference to the accompanying drawings. In the following description, the same reference numerals are assigned to the same components, and repeated description is omitted. In addition, the accompanying drawings are only schematic diagrams, and the size ratio of components to each other, the shape of components, and the like may be different from actual ones.

It should be understood that the terms "include" and "have" in the present disclosure, and any variations thereof, for example, processes, methods, systems, products, or devices that include or have a series of steps or units, are not necessarily limited to those steps or units clearly listed, but may include or have other steps or units not clearly listed or inherent to such processes, methods, products, or devices.

The present disclosure provides a unitary press-triggered applicator, which is an applicator for applying a medical device to a subject (hereinafter may be referred to as the applicator for short). In some examples, the medical device may be a Continuous Glucose Monitoring (CGM) device, and the subject may be a human body. The applicator of the present disclosure provides improved actuation convenience of the triggering mode. In the present disclosure, the triggering mode may refer to an action of triggering the applicator to apply the medical device to the skin surface of the subject.

Hereinafter, the applicator according to the present disclosure will be described by taking a medical device as a continuous glucose monitoring device as an example.

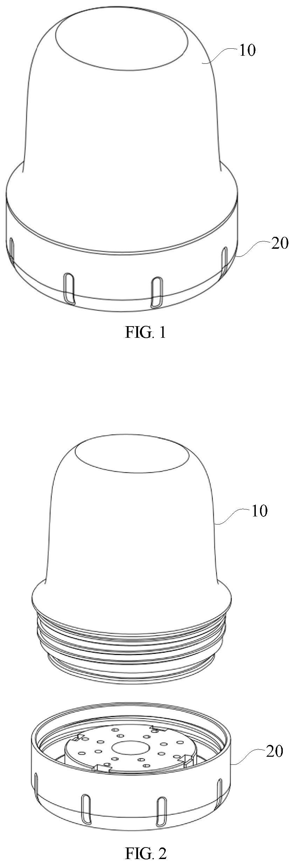

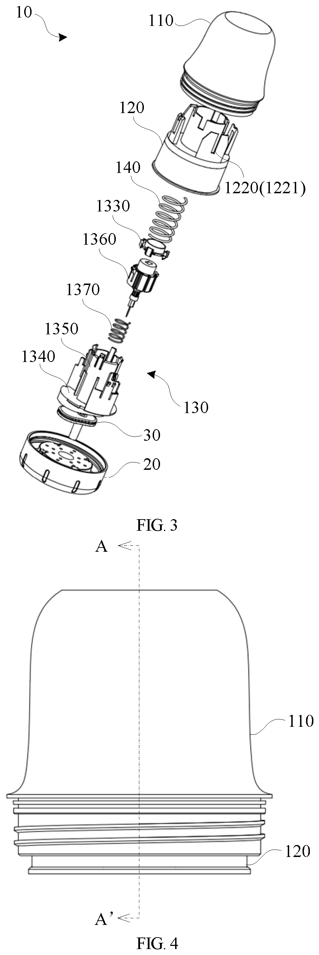

FIG. 1 is a schematic diagram illustrating an assembly of an applicator 10 and a cartridge assembly 20 according to an example of the present disclosure. FIG. 2 is a schematic diagram illustrating separation of an applicator 10 and a cartridge assembly 20 according to an example of the present disclosure. FIG. 3 is an exploded schematic diagram of an applicator 10 and a cartridge assembly 20 according to an example of the present disclosure.

In some examples, the applicator 10 may be assembled to the cartridge assembly 20 (see FIG. 1). In some examples, the applicator 10 may be assembled to the cartridge assembly 20 in a threaded manner. For example, the applicator 10 and the cartridge assembly 20 may respectively have corresponding threads, and the applicator 10 is screwed into the cartridge assembly 20 via the threads.

In some examples, the applicator 10 may be separated from the cartridge assembly 20 (see FIG. 2). In some examples, the applicator 10 may be separated from the cartridge assembly 20 in a threaded manner. For example, the applicator 10 and the cartridge assembly 20 may respectively have corresponding threads, and the applicator 10 is screwed out of the cartridge assembly 20 via the threads.

In some examples, the cartridge assembly 20 may accommodate a medical device 30. In some examples, after the cartridge assembly 20 is assembled to the applicator 10, the applicator 10 may pick up the medical device 30.

In some examples, the applicator 10 may include a proximal end adjacent to the subject during operation and a distal end away from the subject during operation. In other words, in the present disclosure, the "proximal end" refers to an end, relatively close to the subject, of the applicator 10 during operation, the "distal end" refers to an end, relatively far from the subject, of the applicator 10 during operation, and "operation" refers to an operation of applying the medical device 30 to the subject via the applicator 10.

In some examples, the applicator 10 may include a first housing 110 and a second housing 120 (see FIG. 3). The first housing 110 may be assembled to the second housing 120. In some examples, the first housing 110 may be assembled to the second housing 120 by engaging an inner wall of the first housing 110 with an outer periphery of the second housing 120.

In some examples, the first housing 110 is movable relative to the second housing 120 from the distal end toward the proximal end. In the present disclosure, the operation of applying the medical device 30 to the subject via the applicator 10 may refer to an operation of movement of the first housing 110 relative to the second housing 120 from the distal end toward the proximal end after the applicator 10 picks up the medical device 30.

In some examples, after the first housing 110 and the second housing 120 are assembled, the second housing 120 may protrude toward the proximal end beyond the first housing 110. In some examples, a space for the first housing 110 to move relative to the second housing 120 from the distal end toward the proximal end may be formed inside the applicator 10.

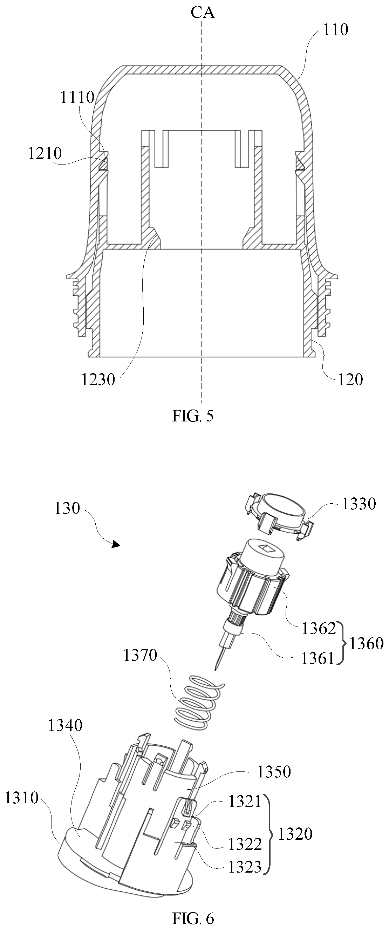

FIG. 4 is a schematic diagram illustrating an assembly of the first housing 110 and the second housing 120 according to an example of the present disclosure. FIG. 5 is a cross-sectional view taken along section line AA' in FIG. 4 according to an example of the present disclosure. A dashed line CA in FIG. 5 schematically depicts a central axis of the first housing 110 or the second housing 120.

Referring to FIG. 5, in some examples, a first limiting mechanism 1110 may be formed on the first housing 110. In some examples, a mating portion 1210 slidably engaged with the first limiting mechanism 1110 may be formed on the second housing 120, such that movement of the first housing 110 relative to the second housing 120 from the distal end toward the proximal end can be facilitated.

In some examples, the first limiting mechanism 1110 may be formed on an inner wall of the first housing 110. The mating portion 1210 may be formed on an outer periphery of the second housing 120.

In some examples, the first limiting mechanism 1110 may include a first inclined surface that gradually approaches a central axis of the first housing 110 in a direction from the proximal end toward the distal end. In some examples, the mating portion 1210 may include a second inclined surface that gradually approaches a central axis of the second housing 120 in the direction from the proximal end toward the distal end. The first inclined surface and the second inclined surface may be matched, such that operation convenience of movement of the first housing 110 relative to the second housing 120 from the distal end toward the proximal end can be improved.

In the present disclosure, the first inclined surface being matched with the second inclined surface may mean that the first inclined surface is attached to the second inclined surface.

In some examples, the first limiting mechanism 1110 may include a first planar surface extending along the inner wall of the first housing 110 toward the central axis of the first housing 110. The first planar surface may be closer to the distal end than the first inclined surface. In some examples, the mating portion 1210 may include a second planar surface extending along the central axis of the second housing 120 toward the outer periphery of the second housing 120. The second planar surface may be closer to the proximal end than the second inclined surface. In some examples, the first planar surface and the second planar surface may be matched. In this case, after the first housing 110 is moved relative to the second housing 120 from the distal end toward the proximal end, the matching between the first inclined surface of the first limiting mechanism 1110 and the second inclined surface of the mating portion 1210 can be switched to the matching between the first planar surface of the first limiting mechanism 1110 and the second planar surface of the mating portion 1210, and the possibility that the second housing 120 and the first housing 110 return to the state where the first inclined surface is matched with the second inclined surface to allow re-use of the applicator 10 can be reduced.

In the present disclosure, the first planar surface being matched with the second planar surface may mean that the first planar surface is attached to the second planar surface.

In some examples, the number of the first limiting mechanisms 1110 may be two. The two first limiting mechanisms 1110 may be located on the same side of the central axis of the first housing 110. In some examples, the mating portion 1210 may be disposed between the two first limiting mechanisms 1110, such that assembly stability of the first housing 110 and the second housing 120 can be improved.

In some examples, the number of the first limiting mechanisms 1110 may be four. The four first limiting mechanisms 1110 may be respectively located on two sides of the central axis of the first housing 110 in pairs.

In some examples, the applicator 10 may include a movable assembly 130 (see FIG. 3). The movable assembly 130 may be assembled to the second housing 120. In some examples, the movable assembly 130 may be assembled to the second housing 120 by fitting an outer periphery of the movable assembly 130 with an inner wall of the second housing 120.

In some examples, the movable assembly 130 is movable relative to the second housing 120 from the distal end toward the proximal end.

In some examples, the movable assembly 130 may be at least partially disposed between the first housing 110 and the second housing 120, such that structural compactness of the applicator 10 can be improved.

In some examples, when the first housing 110 moves relative to the second housing 120 from the distal end toward the proximal end, the movable assembly 130 may be configured to be movable toward the proximal end.

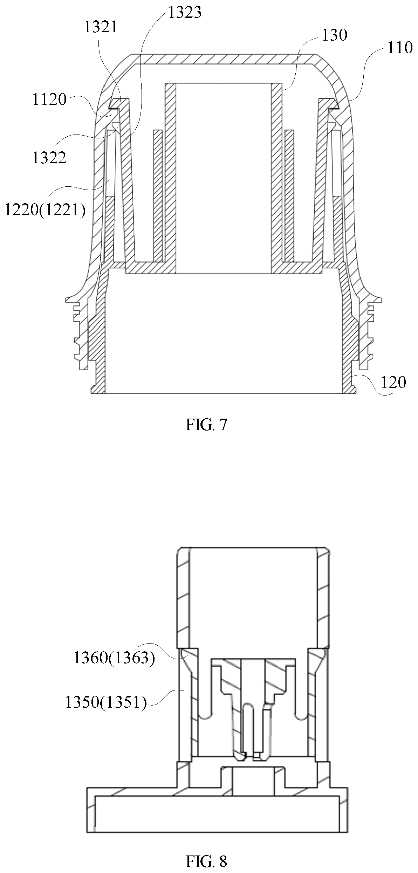

FIG. 6 is an exploded schematic diagram of a movable assembly 130 according to an example of the present disclosure. FIG. 7 is a cross-sectional view of an applicator 10 taken along a plane perpendicular to section line AA' in FIG. 4 according to an example of the present disclosure. To better illustrate the fitting position between the retaining mechanism 1320 and the first housing 110 and the second housing 120, some components or lines that may affect the description are omitted and simplified in FIG. 7.

In some examples, the movable assembly 130 may include a receiving portion 1310 for accommodating the medical device 30 (see FIG. 6). The receiving portion 1310 may have a receiving space adapted to an outer contour of the medical device 30. In this case, when the movable assembly 130 moves toward the proximal end, the movable assembly 130 can drive the medical device 30 to move toward the proximal end, thereby improving operation convenience of applying the medical device 30 to the subject.

In some examples, the movable assembly 130 may include a retaining mechanism 1320 releasably retained by the first housing 110 and the second housing 120 (see FIG. 6 and FIG. 7). In other words, with the cooperation of the retaining mechanism 1320 with the first housing 110 and the second housing 120, the movable assembly 130 may be configured to be retained by the first housing 110 and the second housing 120.

In some examples, when the first housing 110 moves relative to the second housing 120 from the distal end toward the proximal end, the retaining mechanism 1320 may be released from the first housing 110 and the second housing 120, such that the movable assembly 130 is configured to be movable toward the proximal end.

In some examples, the applicator 10 may further include a first driving mechanism 140 (see FIG. 3). The first driving mechanism 140 may be disposed between the first housing 110 and the movable assembly 130. In some examples, the first driving mechanism 140 may apply to the movable assembly 130 an acting force toward the proximal end. In this case, after the first housing 110 and the second housing 120 release the retaining mechanism 1320, the acting force applied by the first driving mechanism 140 to the movable assembly 130 can drive the movable assembly 130 to move toward the proximal end, thereby facilitating proximal movement of the medical device 30 by the movable assembly 130.

In some examples, the first driving mechanism 140 may be a spring in a compressed state. In other examples, the first driving mechanism 140 may also be a spring in a stretched state.

In some examples, the retaining mechanism 1320 may include a first retaining portion 1321 (see FIG. 6 and FIG. 7). In some examples, a second limiting mechanism 1120 may be formed on the first housing 110 (see FIG. 7). The first retaining portion 1321 may be releasably retained by the second limiting mechanism 1120, such that the retaining mechanism 1320 can be releasably retained by the first housing 110.

In some examples, when the first housing 110 moves relative to the second housing 120 from the distal end toward the proximal end, the first housing 110 and the second housing 120 may release the retaining mechanism 1320.

In some examples, the first retaining portion 1321 may include a first retaining surface extending along a central axis of the movable assembly 130 toward an outer periphery of the movable assembly 130 (see FIG. 7). In some examples, the second limiting mechanism 1120 may include a second retaining surface extending from an inner wall of the first housing 110 toward the central axis of the first housing 110. In some examples, when the first retaining portion 1321 is releasably retained by the first housing 110, the first retaining surface may abut against the second retaining surface, such that stability when the first retaining portion 1321 is retained by the second limiting mechanism 1120 can be improved.

In some examples, the retaining mechanism 1320 may include a second retaining portion 1322 releasably retained by the second housing 120 (see FIG. 6 and FIG. 7). In other words, the second retaining portion 1322 may be releasably retained by the second housing 120.

In some examples, when the first housing 110 moves relative to the second housing 120 from the distal end toward the proximal end, the second housing 120 may release the second retaining portion 1322.

In some examples, the second retaining portion 1322 may include a second inclined surface that gradually moves away from the central axis of the movable assembly 130 in the direction from the proximal end toward the distal end. In some examples, the second inclined surface may abut against the second housing 120. In this case, when the movable assembly 130 is acted on toward the proximal end, the second inclined surface can gradually disengage from the second housing 120, thereby enabling the second housing 120 to release the second retaining portion 1322.

In some examples, the retaining mechanism 1320 may include a first retaining arm 1323 that flares outwardly in the direction from the proximal end toward the distal end (see FIG. 6 and FIG. 7). The second retaining portion 1322 and the first retaining portion 1321 may be sequentially formed on the first retaining arm 1323 in the direction from the proximal end toward the distal end. In this case, when the second retaining portion 1322 shifts toward the central axis of the movable assembly 130 (for example, when the second retaining portion 1322 is subjected to an acting force toward the central axis of the movable assembly 130), the shifted second retaining portion 1322 can shift the first retaining arm 1323 toward the central axis of the movable assembly 130, such that the shifted first retaining arm 1323 drives the first retaining portion 1321 to shift toward the central axis of the movable assembly 130, thereby enabling the second limiting mechanism 1120 to release the first retaining portion 1321, and facilitating releasing the retaining mechanism 1320 by the first housing 110 and the second housing 120.

In some examples, the first retaining arm 1323 may be formed on an outer periphery of the movable assembly 130.

In some examples, the second housing 120 may include a third limiting mechanism 1220 abutting against the second retaining portion 1322 (see FIG. 3 or FIG. 7). Specifically, when the second retaining portion 1322 is releasably retained by the second housing 120, the second retaining portion 1322 may abut against the third limiting mechanism 1220.

In some examples, after the first retaining portion 1321 is released from the first housing 110, the first retaining portion 1321 may abut against the third limiting mechanism 1220, such that the risk that the released movable assembly 130 is detached from the second housing 120 can be reduced.

In some examples, the third limiting mechanism 1220 may include a limiting notch 1221 recessed in a direction from the distal end toward the proximal end (see FIG. 3 or FIG. 7). In some examples, after the first retaining portion 1321 is released from the first housing 110, the first retaining surface may abut against the limiting notch 1221.

In some examples, the movable assembly 130 may have a first bottom portion 1330 proximate to the distal end (see FIG. 3 or FIG. 6). In some examples, the first driving mechanism 140 may be disposed between the first housing 110 and the first bottom portion 1330, such that the first driving mechanism 140 can act on the movable assembly 130 by acting on the first bottom portion 1330.

In some examples, the movable assembly 130 may have an opening proximate to the distal end. Specifically, the movable assembly 130 may not include the first bottom portion 1330, and an end, proximate to the distal end, of the movable assembly 130 may be provided as an opening.

In some examples, the movable assembly 130 may have a second bottom portion 1340 proximate to the proximal end (see FIG. 3 or FIG. 6). The second bottom portion 1340 may be connected to an upper surface of the receiving portion 1310.

In some examples, the movable assembly 130 may have a side wall 1350 connecting the first bottom portion 1330 and the second bottom portion 1340 (see FIG. 3 or FIG. 6), such that structural compactness of the movable assembly 130 can be improved.

In some examples, the side wall 1350 may connect the opening and the second bottom portion 1340, such that structural flexibility of the movable assembly 130 can be improved.

In some examples, the side wall 1350 may include an extension section extending along the central axis of the movable assembly 130 toward the outer periphery of the movable assembly 130. In some examples, the first driving mechanism 140 may be disposed between the first housing 110 and the extension section, such that by acting on the extension section, the first driving mechanism 140 can act on the movable assembly 130.

In some examples, the movable assembly 130 may include a guide member 1360 for guiding application of the medical device 30 (see FIG. 3 or FIG. 6). Specifically, the guide member 1360 may be coupled with the medical device 30, and when the first housing 110 moves relative to the second housing 120 from the distal end toward the proximal end, the guide member 1360 may guide the medical device 30 to be applied to the subject.

In some examples, the guide member 1360 may have a piercing element 1361 coupled with the medical device 30 (see FIG. 6), such that convenience of partially implanting the medical device 30 under the skin of the subject can be improved.

In some examples, the guide member 1360 may have a clamping holder 1362 for fixing the piercing element 1361 (see FIG. 6).

In some examples, the side wall 1350, the first bottom portion 1330 and the second bottom portion 1340 may form an accommodating space in which the guide member 1360 is configured to move. In some examples, the medical device 30 may be disposed in the accommodating space, such that the guide member 1360 is not easily detached from the accommodating space.

FIG. 8 is a schematic cross-sectional view illustrating cooperation between a guide member 1360 and a side wall 1350 according to an example of the present disclosure. To better illustrate the fitting position between the guide member 1360 and the side wall 1350, some components or lines that may affect the illustration are omitted and simplified in FIG. 8.

In some examples, the guide member 1360 may be releasably retained by the side wall 1350.

In some examples, a second driving mechanism 1370 may be disposed between the guide member 1360 and the second bottom portion 1340 (see FIG. 3 or FIG. 6). In other words, the movable assembly 130 may further include a second driving mechanism 1370, and the second driving mechanism 1370 may be disposed between the guide member 1360 and the second bottom portion 1340. In some examples, the second driving mechanism 1370 may apply to the guide member 1360 an acting force toward the distal end. In this case, when the guide member 1360 is released from the side wall 1350, the acting force applied by the second driving mechanism 1370 to the guide member 1360 can drive the guide member 1360 to move toward the distal end, such that the guide member 1360 can be conveniently decoupled from the medical device 30 after the guide member 1360 guides the medical device 30 to be applied to the subject, thereby improving operation convenience of the applicator 10.

In some examples, the second driving mechanism 1370 may be a spring in a compressed state. In other examples, the second driving mechanism 1370 may also be a spring in a stretched state.

In some examples, the guide member 1360 may include a second retaining arm 1363 (see FIG. 8). The second retaining arm 1363 may be releasably retained by the side wall 1350.

In some examples, the second retaining arm 1363 may be formed on an outer periphery of the clamping holder 1362.

In some examples, the second retaining arm 1363 may flare outwardly from the proximal end toward the distal end. A free end of the second retaining arm 1363 may abut against the side wall 1350.

In some examples, the side wall 1350 may include a retaining notch 1351 fitted with the second retaining arm 1363 (see FIG. 8). In some examples, the second retaining arm 1363 may abut against the retaining notch 1351, such that the second retaining arm 1363 can be releasably retained by the side wall 1350.

In some examples, a triggering mechanism 1230 may be formed on the second housing 120 (see FIG. 5). In some examples, during movement of the movable assembly 130 toward the proximal end, the triggering mechanism 1230 may act on the second retaining arm 1363. Specifically, during movement of the guide member 1360 toward the proximal end, the triggering mechanism 1230 may act on the free end of the second retaining arm 1363. In this case, the triggering mechanism 1230 acting on the second retaining arm 1363 can shift the second retaining arm 1363 toward the central axis of the movable assembly 130, such that the second retaining arm 1363 disengages from the side wall 1350, thereby enabling the guide member 1360 to move toward the distal end under the action applied by the second driving mechanism 1370, and facilitating decoupling the guide member 1360 from the medical device 30.

In some examples, the triggering mechanism 1230 may be formed on an inner wall of the second housing 120. In some examples, the triggering mechanism 1230 may be a protruding structure formed on the inner wall of the second housing 120.

In the present disclosure, "unitary press-triggered" refers to pressing the first housing 110 toward the proximal end, such that the applicator 10 triggers the action of applying the CGM device to the skin surface of the human body.

In a preferred embodiment of the present disclosure, the applicator 10 may have an initial state before triggering the application action. In the initial state, the second housing 120 protrudes toward the proximal end beyond the first housing 110, and the first housing 110 is movable relative to the second housing 120 from the distal end toward the proximal end; the first retaining portion 1321 and the second retaining portion 1322 of the retaining mechanism 1320 are releasably retained by the second limiting mechanism 1120 of the first housing 110 and the third limiting mechanism 1220 of the second housing 120, respectively; the second retaining arm 1363 of the guide member 1360 is releasably retained by the side wall 1350; the first driving mechanism 140 and the second driving mechanism 1370 are configured to be in a compressed state.

In a preferred embodiment of the present disclosure, when the applicator 10 is in the initial state, pressing the first housing 110 toward the proximal end will cause the first housing 110 to move relative to the second housing 120 from the distal end toward the proximal end. Since the first retaining portion 1321 is releasably retained by the second limiting mechanism 1120, the movable assembly 130 and the first housing 110 will initially remain relatively stationary under the action of the first driving mechanism 140, and at the same time, the movable assembly 130 will move toward the proximal end relative to the second housing 120.

In a preferred embodiment of the present disclosure, during the movement of the movable assembly 130 toward the proximal end, the third limiting mechanism 1220 gradually disengages from the second retaining portion 1322, and the third limiting mechanism 1220 gradually increases the acting force applied to the second retaining portion 1322 toward the central axis of the movable assembly 130, such that the first retaining arm 1323 shifts toward the central axis of the movable assembly 130. The shifted first retaining arm 1323 drives the first retaining portion 1321 to shift toward the central axis of the movable assembly 130, such that the third limiting mechanism 1220 releases the second retaining portion 1322 and the second limiting mechanism 1120 releases the first retaining portion 1321, whereby the first housing 110 and the second housing 120 release the movable assembly 130 (that is, the released movable assembly 130 moves relative to both the first housing 110 and the second housing 120).

In a preferred embodiment of the present disclosure, the released movable assembly 130 will move toward the proximal end under the action of the first driving mechanism 140 and guide the CGM device to be applied to the skin surface of the human body. When the movable assembly 130 moves to a designed position (that is, when the movable assembly 130 moves to a position where the triggering mechanism 1230 acts on the second retaining arm 1363), the second retaining arm 1363 disengages from the side wall 1350 such that the guide member 1360 is released by the side wall 1350. The released guide member 1360 will move toward the distal end under the action of the second driving mechanism 1370 and be decoupled from the medical device 30.

In the present disclosure, movement of the first housing 110 relative to the second housing 120 from the distal end toward the proximal end enables the first housing 110 and the second housing 120 to release the retaining mechanism 1320. In other words, pressing the first housing 110 enables the movable assembly 130 to move relative to the second housing 120 from the distal end toward the proximal end to guide the medical device 30 to be applied to the subject, thereby providing improved actuation convenience of the triggering mode.

Although the present disclosure has been specifically described above with reference to the accompanying drawings and examples, it should be understood that the above description does not limit the present disclosure in any form. A person skilled in the art can make modifications and variations to the present disclosure as required without departing from the essential spirit and scope of the present disclosure, and such modifications and variations all fall within the scope of the present disclosure.

## Claims

1. A unitary press-triggered applicator for applying a medical device to a subject, comprising: a first housing, a second housing, a movable assembly, a proximal end adjacent to the subject during operation, and a distal end away from the subject during operation, wherein the first housing is assembled to the second housing and the movable assembly is assembled to the second housing, the movable assembly, which is movable relative to the second housing from the distal end toward the proximal end, comprises a receiving portion configured to accommodate the medical device, a retaining mechanism releasably retained by the first housing and the second housing, and a guide member configured to guide application of the medical device; and the first housing and the second housing release the retaining mechanism when the first housing moves relative to the second housing from the distal end toward the proximal end.

2. The unitary press-triggered applicator according to claim 1, further comprising a first driving mechanism disposed between the first housing and the movable assembly, wherein the first driving mechanism applies to the movable assembly an acting force toward the proximal end.

3. The unitary press-triggered applicator according to claim 1, wherein the first housing defines a first limiting mechanism, and the second housing defines a mating portion slidably engaged with the first limiting mechanism.

4. The unitary press-triggered applicator according to claim 1, wherein the retaining mechanism comprises a first retaining portion, the first housing defines a second limiting mechanism, and the first retaining portion is releasably retained by the second limiting mechanism.

5. The unitary press-triggered applicator according to claim 4, wherein the retaining mechanism comprises a first retaining arm that flares outwardly in a direction from the proximal end toward the distal end, and a second retaining portion releasably retained by the second housing; the second retaining portion and the first retaining portion are sequentially formed on the first retaining arm in the direction from the proximal end toward the distal end.

6. The unitary press-triggered applicator according to claim 5, wherein the second housing comprises a third limiting mechanism abutting against the second retaining portion, and the first retaining portion abuts against the third limiting mechanism after the first retaining portion is released from the first housing.

7. The unitary press-triggered applicator according to claim 2, wherein the movable assembly has a first bottom portion proximate to the distal end, a second bottom portion proximate to the proximal end, and a side wall connecting the first bottom portion and the second bottom portion; the first driving mechanism is disposed between the first housing and the first bottom portion.

8. The unitary press-triggered applicator according to claim 2, wherein the movable assembly has an opening proximate to the distal end, a second bottom portion proximate to the proximal end, and a side wall connecting the opening and the second bottom portion; the side wall comprises an extension section extending along a central axis of the movable assembly toward an outer periphery of the movable assembly; the first driving mechanism is disposed between the first housing and the extension section.

9. The unitary press-triggered applicator according to claim 7, wherein the guide member is releasably retained by the side wall, a second driving mechanism is disposed between the guide member and the second bottom portion, and the second driving mechanism applies to the guide member an acting force toward the distal end.

10. The unitary press-triggered applicator according to claim 9, wherein the guide member comprises a second retaining arm releasably retained by the side wall; the second retaining arm flares outwardly in a direction from the proximal end toward the distal end and abuts against the side wall; the second housing defines a triggering mechanism, and the triggering mechanism acts on the second retaining arm during movement of the movable assembly toward the proximal end.
